(19) Europäisches Patentamt
European Patent Office
Office européen des brevets

(11) Veröffentlichungsnummer: **0 011 182**
**A2**

# (12) EUROPÄISCHE PATENTANMELDUNG

(21) Anmeldenummer: **79104241.9**

(22) Anmeldetag: **31.10.79**

(51) Int. Cl.³: **C 07 C 127/19**
C 07 C 149/437, A 01 N 47/34
//C07C123/00

(30) Priorität: **15.11.78 DE 2849558**

(43) Veröffentlichungstag der Anmeldung:
**28.05.80 Patentblatt 80/11**

(84) Benannte Vertragsstaaten:
**BE CH DE FR GB IT NL**

(71) Anmelder: **BAYER AG**
**Zentralbereich Patente, Marken und Lizenzen**
**D-5090 Leverkusen 1, Bayerwerk(DE)**

(72) Erfinder: **Fest, Christa, Dr.**
**Im Johannistal 20**
**D-5600 Wuppertal 1(DE)**

(72) Erfinder: **Kraus, Peter, Dr.**
**Düsseldorferstrasse 43**
**D-5000 Köln 80(DE)**

(72) Erfinder: **Scheinpflug, Hans, Dr.**
**Am Thelenhof 15**
**D-5090 Leverkusen(DE)**

## (54) Substituierte Phenyliminomethyl-harnstoffe, Verfahren zu ihrer Herstellung und ihre Verwendung als Pflanzenbakterizide.

(57) Die Erfindung betrifft neue substituierte Phenyliminomethylharnstoffe, ein Verfahren zu ihrer Herstellung sowie ihre Verwendung als Pflanzenbakterizide.

Die neuen Verbindungen der Formel

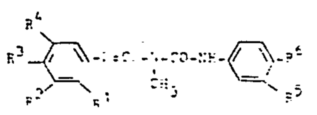

(I)

in welcher $R^1$ bis $R^6$ die in der Beschreibung angegebene Bedeutung besitzen, werden erhalten, wenn man substituierte Phenyl-formamidine mit substituierten Phenylisocyanaten gegebenenfalls in Gegenwart eines inerten Verdünnungsmittels umsetzt.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

EP 0 011 182 A2

BAYER AKTIENGESELLSCHAFT 5090 Leverkusen, Bayerwerk

Zentralbereich
Patente, Marken und Lizenzen

Slr-by-w
Ia

# Substituierte Phenyliminomethyl-harnstoffe, Verfahren zu ihrer Herstellung und ihre Verwendung als Pflanzenbakterizide

Die vorliegende Erfindung betrifft neue substituierte Phenyliminomethylharnstoffe, ein Verfahren zu ihrer Herstellung sowie ihre Verwendung als Pflanzenbakterizide.

Es ist bekannt, daß bestimmte Phenyliminomethyl-harnstoffe, wie z.B. N-(4-Chloro-2-methyl-phenyl-iminomethyl)-N-methyl-N'-phenyl-harnstoff, zur Bekämpfung tierischer und mikrobieller Pflanzenschädlinge verwendet werden können (vgl. hierzu die US-PS 3 960 947 bzw. die DE-OS 2 202 034). Weiter ist bekannt, daß bestimmte Kupferverbindungen, wie z.B. Kupferoxychlorid, fungizide und bakterizide Eigenschaften aufweisen. Die Wirkung dieser bekannten Bakterizide ist jedoch, insbesondere bei niedrigen Wirkstoffkonzentrationen und Aufwandmengen, nicht immer zufriedenstellend.

Es wurden nun neue Phenyliminomethyl-harnstoffe der Formel

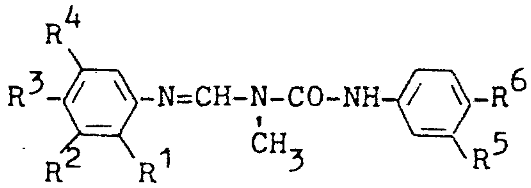

(I)

gefunden, in welcher

$R^1$ für Halogenalkyl und - mit der Maßgabe, daß $R^3$ für Wasserstoff steht - auch für Alkyl oder Halogen steht,

$R^2$ für Wasserstoff oder zusammen mit $R^1$ für Alkandiyl steht,

$R^3$ für Wasserstoff und - mit der Maßgabe, daß $R^1$ für Halogenalkyl steht - auch für Halogen steht,

$R^4$ für Wasserstoff, Alkyl und - mit der Maßgabe, daß $R^3$ für Wasserstoff steht - auch für Halogen steht,

$R^5$ für Wasserstoff oder Halogen steht, und

$R^6$ für Alkyl, Halogen, Halogenalkyl, Alkoxy, Halogenalkoxy, Alkylthio oder Halogenalkylthio steht.

Man erhält die Phenyliminomethyl-harnstoffe der Formel (I), wenn man substituierte Phenyl-formamidine der Formel

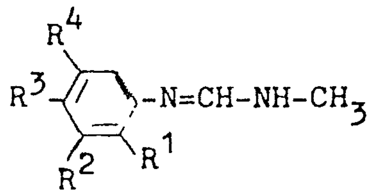

(II)

in welcher die Reste $R^1$ bis $R^4$ die oben angegebene Bedeutung haben,

mit substituierten Phenyl-isocyanaten der Formel

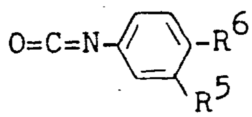

(III)

in welcher die Reste $R^5$ und $R^6$ die oben angegebene Bedeutung haben,

gegebenenfalls in Gegenwart eines inerten Verdünnungsmittels umsetzt.

Die neuen Verbindungen der Formel (I) zeichnen sich durch hohe Wirksamkeit gegen pflanzenschädigende Bakterien aus und sind daher als Pflanzenschutzmittel von Interesse.

Überraschenderweise zeigen die erfindungsgemäßen Phenyliminomethyl-harnstoffe (I) eine erheblich höhere bakterizide Wirkung als die aus dem Stand der Technik bekannten Verbindungen analoger Struktur und/oder gleicher Wirkungsrichtung.

Die Erfindung betrifft vorzugsweise Verbindungen der Formel (I), in welcher

$R^1$ für Trifluormethyl und - mit der Maßgabe, daß $R^3$ für Wasserstoff steht - auch für geradkettiges oder verzweigtes Alkyl mit 1 bis 4 Kohlenstoffatomen, wie insbesondere für Methyl, oder für Chlor oder Brom steht,

$R^2$ für Wasserstoff oder zusammen mit $R^1$ für geradkettiges $\alpha,\omega$-Alkandiyl mit 2 bis 6 Kohlenstoffatomen, das heißt somit für die Gruppe $-(CH_2)_n-$ steht, wobei der Index n für ganze Zahlen von 2 bis 6 und insbesondere für die Zahl 4 steht,

$R^3$ für Wasserstoff und - mit der Maßgabe, daß $R^1$ für Trifluormethyl steht - auch für Chlor oder Brom steht,

$R^4$ für Wasserstoff, geradkettiges oder verzweigtes Alkyl mit 1 bis 4 Kohlenstoffatomen wie insbesondere für Methyl, und - mit der Maßgabe, daß $R^3$ für Wasserstoff steht - auch für Chlor oder Brom steht,

$R^5$ für Wasserstoff, Chlor oder Brom steht, und

$R^6$ für geradkettiges oder verzweigtes Alkyl mit 1 bis 4 Kohlenstoffatomen, wie insbesondere für Methyl, für Chlor oder Brom, geradkettiges oder verzweigtes Alkoxy oder Alkylthio mit jeweils 1 bis 4 Kohlenstoffatomen, insbesondere mit jeweils 1 bis 2 Kohlenstoffatomen, oder für Fluoralkoxy-, Chlorfluoralkoxy, Fluoralkylthio oder Chlorfluoralkylthio mit jeweils 1 oder 2 Kohlenstoffatomen steht.

Im einzelnen seien hier die folgenden wichtigen Verbindungen aufgezählt: von praktischem Interesse sind insbesondere der N-(2,5-Dichlorphenylimino-methyl)-N-methyl-N'-(3-chloro-4-methyl-phenyl)-harnstoff und der N-(2-Trifluormethyl-4-chlorophenylimino-methyl)-N-methyl-N'-(3-chloro-4-trifluormethyl-phenyl)-harnstoff, ferner noch der N-(2-Chloro-5-methyl-phenylimino-methyl)-N-methyl-N'-(4-äthoxyphenyl)-harnstoff, N-(2-Chloro-5-methyl-phenylimino-methyl)-N-methyl-N'-(3-chloro-4-methyl-phenyl)-harnstoff, N-(2-Methyl-phenylimino-methyl)-N-methyl-N'-(3-chloro-4-methyl-phenyl)-harnstoff, N-(Tetrahydronaphthylimino-methyl)-N-methyl-N'-(3,4-dichlorphenyl)-harnstoff, N-(2,5-Dichloro-phenylimino-methyl)-N-methyl-N'-(3-chloro-4-difluorchloromethylthio-phenyl)-harnstoff und der N-(2,5-Dichloro-phenylimino-methyl)-N-methyl-N'-(3-chloro-4-trifluormethoxy-phenyl)-harnstoff.

Verwendet man beispielsweise N-(2,5-Dimethyl-phenyl)-N'-methyl-formamidin und 3-Chloro-4-trifluormethyl-thio-phenylisocyanat als Ausgangsstoffe, so kann der Reaktionsablauf durch das folgende Formelschema skizziert werden:



BAD ORIGINAL

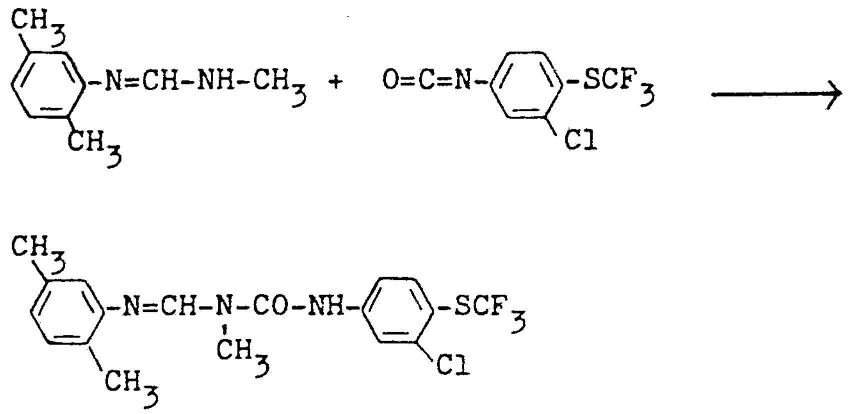

Die als Ausgangsstoffe zu verwendenden Phenyl-formamidine sind durch die Formel (II) definiert.
Vorzugsweise haben darin $R^1$, $R^2$, $R^3$ und $R^4$ die bei der Definition der Reste $R^1$ bis $R^4$ in Formel (I) als bevorzugt angegebene Bedeutung.
Als Beispiele seien genannt:
N-(2-Trifluormethyl-phenyl)-, N-(4-Chloro-2-trifluormethylphenyl)-, N-(2-Methyl-phenyl)-, N-(2,5-Dimethyl-phenyl)-, N-(5-Chloro-2-methyl-phenyl)-, N-(2-Chloro-phenyl)-, N-(2,5-Dichloro-phenyl)-, N-(2-Chloro-5-methyl-phenyl)-, N-(2-Bromo-phenyl)- und N-[1-(5,6,7,8-Tetrahydro)-naphthyl]-N'-methyl-formamidin.

Die Ausgangsverbindungen der Formel (II) sind bekannt oder können analog bekannten Verfahren hergestellt werden (vergleiche DE-OS 2 751 317). So erhält man beispielsweise N-(2,5-Dichloro-phenyl)-N'-methyl-formamidin durch Umsetzung von 2,5-Dichloranilin mit N-Methyl-formamid in Gegenwart von Phosphoroxychlorid bei Temperaturen zwischen 10 und 60 °C. Zur Aufarbeitung wird das Reaktionsgemisch mit Wasser verdünnt, mit Natronlauge alkalisch gestellt und das Produkt durch Filtration isoliert.

Die weiter als Ausgangsstoffe zu verwendenden Phenyl-isocyanate sind durch Formel (III) definiert. Darin stehen vorzugsweise $R^5$ und $R^6$ für diejenigen Substituenten, welche bei der Definition der Reste $R^5$ und $R^6$ in Formel (I) als bevorzugt genannt sind.

Als Beispiele seien im einzelnen genannt:
3-Bromo-, 4-Bromo-, 3-Chloro-, 4-Chloro-, 3,4-Dichloro-, 4-Methyl-, 3-Chloro-4-methyl-, 4-Trifluormethyl-, 3-Chloro-4-trifluormethyl-, 4-Methoxy-, 4-Äthoxy-, 4-Methylthio-, 4-Trifluormethoxy-, 3-Chloro-4-trifluormethoxy-, 4-(1,2,2-Trifluoräthoxy)-, 4-Trifluormethylthio-, 3-Chloro-4-trifluormethylthio-, 4-Chlordifluormethylthio- und 3-Chloro-4-chlordifluormethylthio-phenylisocyanat.

Die Phenylisocyanate der Formel (III) sind bekannt oder können nach literaturbekannten Methoden, beispielsweise durch Umsetzung der entsprechenden Anilinderivate mit Phosgen, hergestellt werden (vergleiche Houben-Weyl, Methoden der organischen Chemie, 4. Aufl., Band 8, S. 12o, Georg-Thieme-Verlag, Stuttgart 1952).

Das Verfahren zur Herstellung der erfindungsgemäßen Phenyliminomethyl-harnstoffe (I) wird bevorzugt unter Verwendung geeigneter Lösungs- oder Verdünnungsmittel durchgeführt. Als solche kommen praktisch alle inerten organischen Solventien infrage. Hierzu gehören insbesondere aliphatische und aromatische, gegebenenfalls chlorierte Kohlenwasserstoffe, wie Benzin, Benzol, Toluol, Xylol, Methylenchlorid, Chloroform, Tetrachlorkohlenstoff, Chlorbenzol

und o-Dichlorbenzol, Äther wie Diäthyl- und Dibutyläther, Tetrahydrofuran und Dioxan, Ketone wie Aceton, Methyläthyl-, Methylisopropyl- und Methylisobutylketon sowie Nitrile wie Acetonitril und Propionitril.

Die Reaktionstemperatur kann innerhalb eines größeren Bereichs variiert werden. Im allgemeinen arbeitet man zwischen 0 und $80^{\circ}C$, vorzugsweise bei 10 bis $50^{\circ}C$.

Das erfindungsgemäße Verfahren wird im allgemeinen bei Normaldruck durchgeführt.

Zur Durchführung des erfindungsgemäßen Verfahrens werden die Ausgangsstoffe gewöhnlich in äquimolaren Mengen eingesetzt. Ein Überschuß der einen oder anderen Reaktionskomponente bringt keine wesentlichen Vorteile. Die Umsetzung wird im allgemeinen in einem geeigneten Verdünnungsmittel durchgeführt und das Reaktionsgemisch wird mehrere Stunden bei der erforderlichen Temperatur gerührt.

Zur Aufarbeitung wird filtriert, das Lösungsmittel abdestilliert und das als Rückstand verbleibende Rohprodukt gegebenenfalls durch Umkristallisation gereinigt. Die neuen Produkte werden durch ihren Schmelzpunkt charakterisiert.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.



BAD ORIGINAL

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylen-

chlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige,

0011182

körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Beispiel

Bakterien-Test / Xanthomonas oryzae

| | | |
|---|---|---|
| Lösungsmittel: | 11,75 Gewichtsteile | Aceton |
| Dispergiermittel: | 0,75 Gewichtsteile | Alkylarylpolyglykoläther |
| Wasser: | 987,50 Gewichtsteile | |
| andere Zusätze: | - Gewichtsteile | - |

Man vermischt die für die gewünschte Wirkstoffkonzentration in der Spritzflüssigkeit nötige Wirkstoffmenge mit der angegebenen Menge des Lösungsmittels und des Dispergiermittels und verdünnt das Konzentrat mit der angegebenen Menge Wasser.

Mit der Spritzflüssigkeit bespritzt man 30 etwa 40 Tage alte Reispflanzen bis zur Tropfnässe. Die Pflanzen verbleiben bis zum Abtrocknen in einem Gewächshaus bei Temperaturen von 22 bis 24°C und einer relativen Luftfeuchtigkeit von etwa 70 %. Danach werden Nadeln in eine wäßrige Bakteriensuspension von Xanthomonas oryzae getaucht und die Pflanzen durch Anstechen der Blätter inokuliert. Die Pflanzen stehen nach der Inokulation 24 Stunden bei 100 % relativer Luftfeuchtigkeit und danach in einem Raum bei 26 bis 28°C und 80 % relativer Luftfeuchtigkeit. 10 Tage nach der Inokulation wird der Befall bei allen durch Stich verletzten, inokulierten Blätter von vorher mit Präparat behandelten Pflanzen in Wertzahlen von 1 bis 9 ausgewertet. 1 bedeutet 100 %ige Wirkung, 3 = gute Wirkung, 5 = mäßige Wirkung und 9 = keine Wirkung.

Bei diesem Test zeigen z.B. die folgenden Verbindungen eine überlegene Wirkung gegenüber dem Stand der Technik:

Verbindungen gemäß Herstellungsbeispielen 1, 2, 3, 6, 4, 5, 7, 8.

Herstellungsbeispiele

Beispiel 1:

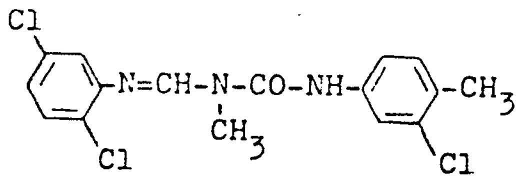

17 g (0,1 Mol) 3-Chloro-4-methyl-phenylisocyanat werden zu einer Lösung von 20 g (0,1 Mol) N-(2,5-Dichloro-phenyl)-N'-methyl-formamidin in 200 ml Methylenchlorid gegeben. Das Reaktionsgemisch wird dann 15 Stunden bei 15 bis 25°C gerührt, filtriert, und durch Einengen im Vakuum vom Lösungsmittel befreit. Nach Umkristallisieren aus iso-Propanol erhält man 32 g (86 % der Theorie) N-(2,5-Dichloro-phenyliminomethyl)-N-methyl-N'-(3-chloro-4-methyl-phenyl)-harnstoff mit einem Schmelzpunkt von 160°C.

Vorprodukt:

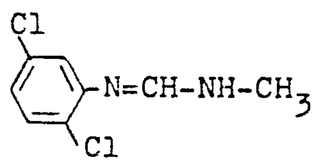

93 ml Phosphoroxychlorid werden zu einer auf 5o°C erwärmten Mischung aus 162 g (1 Mol) 2,5-Dichloroanilin und 5oo ml N-Methyl-formamid gegeben. Das Reaktionsgemisch wird 15 Stunden bei 15 bis 25°C gerührt, dann mit Wasser verdünnt und mit Natronlauge alkalisch gestellt. Das hierbei ausgefallene Produkt wird durch Vakuumfiltration isoliert und durch Umkristallisation aus iso-Propanol gereinigt. Man erhält 94 g (46% der Theorie) N-(2,5-Dichloro-phenyl)-N'-methyl-formamidin mit einem Schmelzpunkt von 91°C.

Analog Beispiel 1 können die folgenden Verbindungen der Formel (I) hergestellt werden:

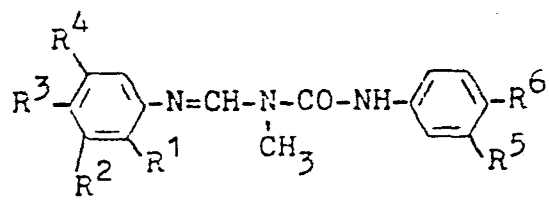

(I)

| Beispiel Nr.: | $R^1$ | $R^2$ | $R^3$ | $R^4$ | $R^5$ | $R^6$ | Schmelzpunkt (°C) | Ausbeute (% der Theorie) |
|---|---|---|---|---|---|---|---|---|
| 2 | $CF_3$ | H | Cl | H | Cl | $CF_3$ | 141 | 61 |
| 3 | Cl | H | H | $CH_3$ | H | $OC_2H_5$ | 45 | 84 |
| 4 | Cl | H | H | $CH_3$ | Cl | $CH_3$ | 144 | 56 |
| 5 | $CH_3$ | H | H | H | Cl | $CH_3$ | 92 | 70 |
| 6 | $-(CH_2)_4-$ | | H | H | Cl | Cl | 115 | 74 |
| 7 | Cl | H | H | Cl | Cl | $SCF_2Cl$ | 140 | 68 |
| 8 | Cl | H | H | Cl | Cl | $OCF_3$ | 113 | 63 |
| 9 | Cl | H | H | H | H | $OC_2H_5$ | 148 | 78 |
| 10 | Cl | H | H | H | Cl | Cl | 111 | 76 |
| 11 | Cl | H | H | H | H | Cl | 84 | 77 |
| 12 | Cl | H | H | H | Cl | $CH_3$ | 120 | 74 |
| 13 | $CF_3$ | H | Cl | H | Cl | $CH_3$ | 145 | 88 |

| Beispiel Nr. | $R^1$ | $R^2$ | $R^3$ | $R^4$ | $R^5$ | $R^6$ | Schmelzpunkt (°C) | Ausbeute (% der Theorie) |
|---|---|---|---|---|---|---|---|---|
| 14 | $CF_3$ | H | Cl | H | Cl | Cl | 151 | 87 |
| 15 | $CF_3$ | H | Cl | H | H | Cl | 128 | 72 |
| 16 | $CF_3$ | H | Cl | H | H | $OC_2H_5$ | 138 | 83 |
| 17 | $CH_3$ | H | H | Cl | Cl | Cl | 153 | 52 |
| 18 | $CH_3$ | H | H | Cl | H | Cl | 140 | 79 |
| 19 | $CH_3$ | H | H | Cl | H | $OC_2H_5$ | 102 | 79 |
| 20 | $CH_3$ | H | H | Cl | Cl | $CH_3$ | 129 | 76 |
| 21 | $CF_3$ | H | Cl | H | H | $CH_3$ | 132 | 78 |
| 22 | $CH_3$ | H | H | Cl | H | $CH_3$ | 130 | 66 |
| 23 | Cl | H | H | Cl | H | $OC_2H_5$ | 136 | 46 |
| 24 | $CH_3$ | H | H | Cl | H | $OC_2H_5$ | 102 | 79 |
| 25 | $CH_3$ | H | H | Cl | Cl | $CH_3$ | 126 | 69 |
| 26 | $CH_3$ | H | H | Cl | Cl | $CF_3$ | 132 | 73 |
| 27 | $CH_3$ | H | H | Cl | H | $CH_3$ | 131 | 86 |
| 28 | Cl | H | H | Cl | Cl | $CF_3$ | 148 | 79 |
| 29 | Cl | H | H | Cl | H | $CH_3$ | 163 | 57 |
| 30 | Cl | H | H | Cl | H | Cl | 174 | 67 |
| 31 | $CF_3$ | H | Cl | H | H | $OC_2H_5$ | 136 | 67 |
| 32 | $CF_3$ | H | Cl | H | Cl | Cl | 153 | 55 |
| 33 | Cl | H | H | $CH_3$ | Cl | Cl | 118 | 81 |

| Beispiel Nr. | $R^1$ | $R^2$ | $R^3$ | $R^4$ | $R^5$ | $R^6$ | Schmelzpunkt (°C) | Ausbeute (% der Theorie) |
|---|---|---|---|---|---|---|---|---|
| 34 | Cl | | H | H | $CH_3$ | H | $CH_3$ | 120 | 75 |
| 35 | Cl | H | H | $CH_3$ | H | Cl | 151 | 54 |
| 36 | $CH_3$ | H | H | H | H | $OC_2H_5$ | 127 | 76 |
| 37 | $CH_3$ | H | H | H | Cl | Cl | Öl | 66 |
| 38 | $CH_3$ | H | H | H | H | $CH_3$ | 91 | 69 |
| 39 | $CH_3$ | H | H | H | H | Cl | Öl | 90 |
| 40 | $CH_3$ | H | H | H | Cl | $CF_3$ | Öl | 74 |
| 41 | $-(CH_2)_4-$ | | H | H | Cl | $CF_3$ | 149 | 64 |
| 42 | $-(CH_2)_4-$ | | H | H | H | $OCF_3$ | 81 | 68 |
| 43 | $-(CH_2)_4-$ | | H | H | Cl | $OCF_3$ | 90 | 68 |
| 44 | $-(CH_2)_4-$ | | H | H | Cl | $SCF_2Cl$ | 100 | 69 |
| 45 | Cl | H | H | Cl | H | $CF_3$ | 180 | 80 |
| 46 | Cl | H | H | Cl | H | $OCF_3$ | 131 | 60 |
| 47 | $CF_3$ | H | Cl | H | Cl | $OCF_3$ | 133 | 70 |
| 48 | $CF_3$ | H | Cl | H | H | $CF_3$ | 116 | 68 |
| 49 | $CF_3$ | H | Cl | H | H | $OCF_3$ | 139 | 52 |
| 50 | $-(CH_2)_4-$ | | H | H | Cl | $CH_3$ | 115 | 65 |
| 51 | $-(CH_2)_4-$ | | H | H | H | Cl | 112 | 62 |
| 52 | $-(CH_2)_4-$ | | H | H | H | $CH_3$ | 147 | 65 |

Patentansprüche

1) Phenyliminomethyl-harnstoffe der allgemeinen Formel

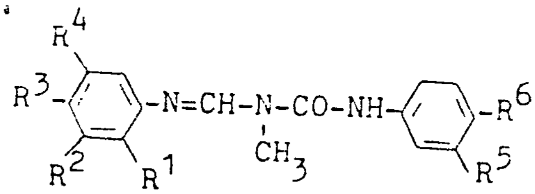

in welcher

$R^1$ für Halogenalkyl und - mit der Maßgabe, daß $R^3$ für Wasserstoff steht - auch für Alkyl oder Halogen steht,

$R^2$ für Wasserstoff oder zusammen mit $R^1$ für Alkandiyl steht,

$R^3$ für Wasserstoff und - mit der Maßgabe, daß $R^1$ für Halogenalkyl steht - auch für Halogen steht,

$R^4$ für Wasserstoff, Alkyl und - mit der Maßgabe, daß $R^3$ für Wasserstoff steht - auch für Halogen steht,

$R^5$ für Wasserstoff oder Halogen steht, und

$R^6$ für Alkyl, Halogen, Halogenalkyl, Alkoxy, Halogenalkoxy, Alkylthio oder Halogenalkylthio steht.

2) Verfahren zur Herstellung von Phenyliminomethyl-harnstoffen, dadurch gekennzeichnet, daß man

0011182

substituierte Phenyl-formamidine der Formel

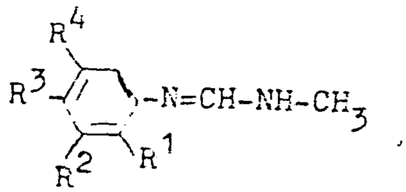

in welcher die Reste $R^1$ bis $R^4$ die in Anspruch 1 angegebene Bedeutung haben, mit substituierten Phenyl-isocyanaten der Formel

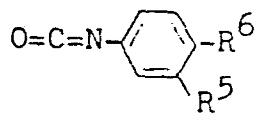

in welcher die Reste $R^5$ und $R^6$ die in Anspruch 1 angegebene Bedeutung haben, gegebenenfalls in Gegenwart eines inerten Verdünnungsmittels umsetzt.

3) Bakterizide Mittel, gekennzeichnet durch einen Gehalt an mindestens einem Phenyliminomethylharnstoff gemäß Anspruch 1.

4) Verfahren zur Bekämpfung von Bakterien, dadurch gekennzeichnet, daß man Phenyliminomethyl-harnstoffe gemäß Anspruch 1 auf Bakterien oder ihren Lebensraum einwirken läßt.

5) Verwendung von Phenyliminomethyl-harnstoffen gemäß Anspruch 1 zur Bekämpfung von Bakterien.

6) Verfahren zur Herstellung von bakteriziden Mitteln, dadurch gekennzeichnet, daß man Phenyliminomethyl-harnstoffe gemäß Anspruch 1 mit Streckmitteln und/oder oberflächenaktiven Mitteln vermischt.